# FASCICULE DE BREVET EUROPEEN

(11) **EP 1 741 402 B1**
(45) Date de publication et mention de la délivrance du brevet: **24.02.2010**
(21) Numéro de dépôt: 06291092.2
(22) Date de dépôt: 03.07.2006
(51) Int. Cl.: A61B 19/00

(54) **Instrument tel qu'un instrument chirurgical**
Vorrichtung wie eine chirurgische Vorrichtung
Instrument such as an surgical instrument

(30) Priorité: 04.07.2005 FR 0507079
(43) Date de publication de la demande: 10.01.2007
(73) Titulaire: De Uthemann, Cyril, 1253 Vandoeuvres (CH)
(72) Inventeur: Despres, Jean-Albert, 41300 Souesmes (FR)
(74) Mandataire: Delaveau, Sophie

(56) Documents cités:
- DE-A1- 3 917 876
- DE-A1- 10 014 542
- GB-A- 2 409 445
- US-A- 5 782 764

## Description

L'invention concerne un instrument tel qu'un instrument chirurgical d'un type prédéterminé, défini par sa forme et sa fonction, parmi une pluralité d'instruments de types différents.

Le document DE 100 14 542 décrit un instrument chirurgical qui comporte un émetteur d'un signal de radio-fréquence, représentatif du code d'identification de l'instrument, qui permet de vérifier si un instrument a pu être oublié dans la plaie d'intervention chirurgicale, avant la fermeture de celle-ci.

Le document US 5782764 a pour objet un instrument médical qui est conçu pour être utilisé dans un système d'imagerie à des fins de diagnostic médical, c'est-à-dire pour visualiser la présence de l'instrument sur une image représentant des tissus d'une partie d'un corps d'un patient.

Le document GB 2409445 décrit un instrument chirurgical qui est identifiable par des marques colorées prévues sur cet instrument.

Les instruments chirurgicaux selon l'état de la technique s'avèrent être complexes ou insuffisant pour une identification sure des instruments.

L'invention a pour but de pallier ces inconvénients.

L'invention comporte les caractéristiques qui sont énoncées dans la partie caractérisante de la revendication 1.

D'autres caractéristiques de l'invention sont indiquées dans des revendications dépendantes.

L'invention sera mieux comprise, et d'autres buts, caractéristiques, détails et avantages de celle-ci apparaîtront plus clairement au cours de la description explicative qui va suivre faite en référence aux dessins schématiques annexés donnés uniquement à titre d'exemple illustrant un mode de réalisation de l'invention et dans lesquels :
- la figure 1 est une vue schématique, en perspective, d'une installation pour le tri des instruments nécessaires à une opération chirurgicale;
- la figure 2 est une vue de dessus d'un plateau de support d'un instrument;
- la figure 3 est une vue éclatée, d'un plateau de support, selon la figure 2 ; et
- les figures 4 et 5 illustrent une configuration avantageuse des plateaux set d'un étage de stockage.

Etant donné que l'avantage de l'invention réside dans le fait que les instruments objets de l'invention permettent une reconnaissance de leur type, à l'aide de rayons X, de façon automatique, on les décrit ci-après dans le cadre d'un procédé et d'une installation de tri automatique d'instruments chirurgicaux. Bien entendu, la description est uniquement donnée à titre d'exemple, mais, de façon générale, l'invention est utilisable à toute opération qui implique une sélection et un rangement d'instruments spécifique pour la mise en oeuvre de l'opération.

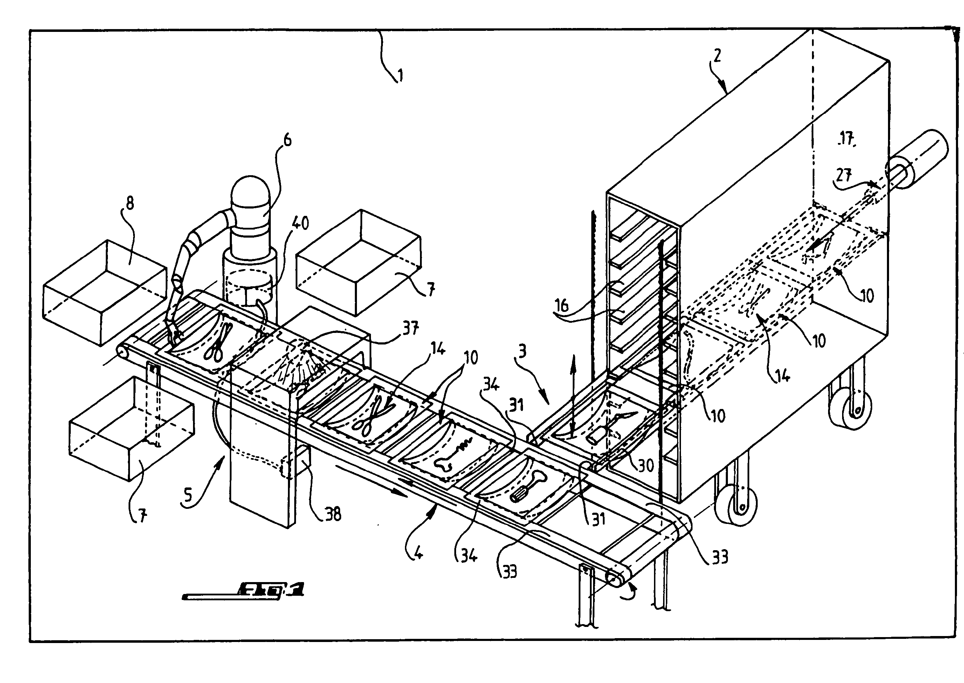

En se reportant à la figure 1, on constate qu'une installation, pour le tri des instruments nécessaires à une opération chirurgicale, comporte essentiellement, disposé à l'intérieur d'une enceinte à ambiance blanche 1, c'est-à-dire d'une propreté la plus parfaite possible, essentiellement un dispositif de stockage de l'ensemble des instruments chirurgicaux susceptibles d'être utilisés pour les différentes opérations chirurgicales possibles, à l'état propre, un dispositif 3 de transfert des instruments à un convoyeur 4 destiné à les transporter à un poste 5 de reconnaissance de la nature ou du type des instruments et un mécanisme de rangement des instruments après leur reconnaissance dans des récipients 7 dont chacun est destiné à contenir les instruments devant être utilisés pour une opération prédéterminée, un récipient 8 étant prévu pour la réception d'instruments jugés non conformes aux exigences établies pour les opérations chirurgicales.

Le dispositif 2 de stockage de l'ensemble des instruments est réalisé, dans le cas d'exemple, sous forme d'un chariot comportant un certain nombre de niveaux 9 de retenue chacun d'une pluralité de plateaux 10, dans l'exemple représenté 3, dont chacun peut comporter une pluralité d'alvéoles 12 de logement d'un instrument chirurgical 14. Dans le cas d'espèce, pour des raisons de simplification des dessins, chaque plateau ne comporte qu'une alvéole. Les plateaux 10 de chaque niveau 9 du chariot de stockage 2 sont supportés par des éléments de support en forme de rail de glissement 16 fixé chacun à une paroi latérale 17 du chariot orienté en direction du convoyeur 4.

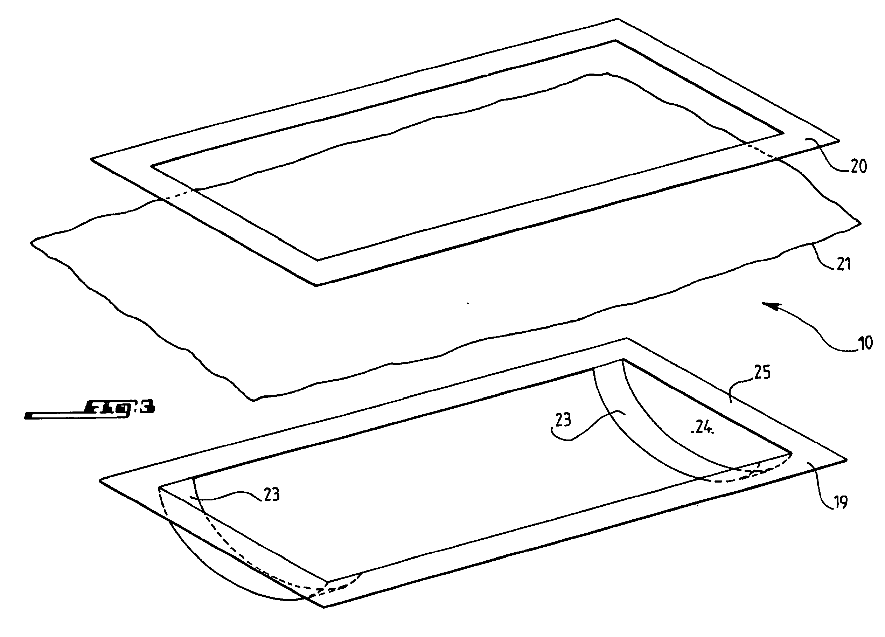

En se reportant à la figure 3, on constate que chaque plateau 10 se compose de deux cadres superposés, en un matériau facilement nettoyable, tel que de l'acier inoxydable, à savoir un élément de cadre inférieur 19 de forme générale rectangulaire et un cadre supérieur 20 de forme complémentaire et susceptible d'être fixé sur le cadre inférieur, par des moyens en forme de clips (non représentés), susceptibles de serrer le cadre supérieur sur le cadre inférieur pour qu'un élément 21 remplaçable, avantageusement à usage unique, en un matériau souple transparent aux rayons X tel que du papier ou du tissu, puisse être inséré à deux de ses bords opposés entre les bords correspondants des deux cadres, pour former une alvéole 12 de logement d'un instrument 14 et ainsi le fond du plateau. Pour la formation aisée des alvéoles et leur maintien, le cadre inférieur 19 est pourvu d'éléments de support 23 en forme d'arcs, au niveau de chaque extrémité longitudinale. Il est avantageux que l'alvéole soit fermée à chaque extrémité longitudinale par une paroi verticale 24 formant l'espace entre le cadre et l'arc correspondant 23.

Comme on le voit sur la figure 1, chaque plateau 10 est déplaçable dans le chariot 2, perpendiculairement à son axe longitudinal en prenant appui par les côtés courts 25 sur les rails de glissement 16 du chariot.

L'agencement de transfert des plateaux 10 de chaque étage du chariot comporte, pour pousser les plateaux hors du chariot, un dispositif poussoir 27 et monté verticalement mobile à l'arrière du chariot 2, pour être positionnable à chaque niveau 9 du chariot. Le dispositif comporte un piston poussoir d'un vérin hydraulique qui, lors de son mouvement de sortie, pousse le dernier plateau et déplace ainsi l'ensemble de plateaux en direction du convoyeur.

Le dispositif de transfert 3 comporte en outre, à l'avant du chariot de stockage 2 de plateaux 10 un cadre 30 de transfert des plateaux 10 du chariot 2 au convoyeur 4, qui est verticalement déplaçable pour pouvoir recevoir les plateaux de chaque niveau 9 de stockage du chariot et pour les transporter ensuite à la hauteur du convoyeur 4 pour que les plateaux puissent être posés sur ce dernier. Plus précisément, dans l'exemple représenté, le cadre de transfert comporte essentiellement deux rails de coulissement 31, chacun étant susceptible d'être aligné, dans une position de réception de plateau d'un niveau ou étage 9, à un rail de glissement 16 de niveau, de façon que les plateaux 10 puissent être déplacés, sous l'effet du dispositif poussoir 27 des rails 16 du chariot aux rails 31 du cadre de transfert 30. Le cadre de transfert est dimensionné de façon à transférer successivement les plateaux 10 au convoyeur 4.

Le convoyeur 4 est montré, sur la figure 1, comme étant réalisé sous forme d'une bande de transport sans fin comportant, essentiellement deux brins plats parallèles 33, destinés au transport des plateaux 10, reliés par des traverses 34.

Pour assurer le transfert des plateaux 10 du cadre de transfert 30 aux brins de convoyeur 33, les rails de support 31 du cadre de transfert s'étendent jusqu'au-dessus du convoyeur et sa portion d'appui de plateau, dans sa position avant de transfert, est abaissable pour permettre la pose des chariots sur les brins du convoyeur situés en dessous. Puis ils s'écartent latéralement et reviennent dans leur position de réception d'un nouveau plateau.

Le convoyeur 4 transporte les plateaux reçus du chariot de stockage 2, par l'intermédiaire du cadre de transfert 3 au poste de reconnaissance 5 des instruments placés dans les alvéoles 12 des plateaux 10.

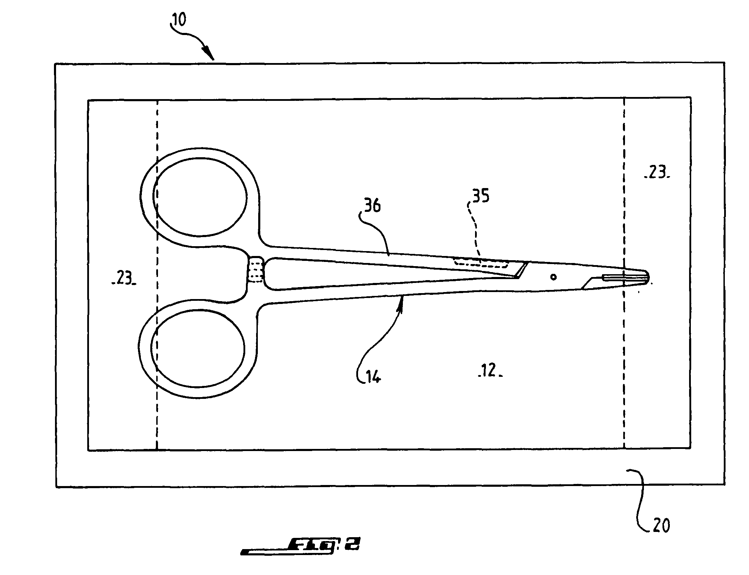

Concernant les instruments chirurgicaux, de fonctions et de formes différentes, ils sont chacun pourvus d'un code d'identité, reconnaissable par le poste 5 de façon que celui-ci soit en mesure de distinguer les instruments apportés par le convoyeur 4 selon leur type spécifique. Plus précisément, le code d'identité de chaque instrument est marqué, sous toute forme appropriée, sur un insert 35 qui est placé dans une cavité appropriée usinée dans l'instrument et fermée ensuite. La figure 2 montre à titre d'exemple, une pince chirurgicale dans une des branches notée 36 de laquelle est incorporé un insert 35.

Le poste de reconnaissance des instruments est avantageusement un lecteur aux rayons X, en forme d'un portique, à travers lequel passe le convoyeur 4 et qui comporte, disposée au-dessus du convoyeur 4, une source de rayons X 37, tandis qu'un récepteur des rayons ayant traversé l'instrument 14 est disposé sous le convoyeur.

Pour pouvoir reconnaître le type d'un instrument, d'après son insert, les éléments de celui-ci, qui constituent le code d'identification, doivent être moins transparents aux rayons X que le matériau constitutif de l'instrument. Ce code pourrait résider par exemple dans la forme de l'insert ou des marques prévues sur celui-ci ou encore la forme ou l'emplacement d'une encoche pratiquée dans l'insert. Des inserts de ce genre étant connus en soi, il n'est pas nécessaire de le décrire plus en détail. Les inserts sont avantageusement réalisés en un matériau relativement opaque aux rayons X. Ils pourraient être faits en laiton ou un alliage à base de laiton, alors que les instruments sont en acier inoxydable. De façon générale, pour le choix des matériaux, plus les atomes constitutifs du matériau sont lourds, c'est-à-dire d'une masse atomique élevée, plus ils absorbent les rayons X, donc plus le matériau est opaque à ces rayons X. Pour assurer une reconnaissance fiable des instruments, il est nécessaire que les instruments soient présentés au poste de reconnaissance dans une position toujours clairement définie.

Après la reconnaissance des instruments, par lecture de leurs inserts, à l'aide des rayons X, l'appareil de rangement 6 saisit les instruments et les range dans les récipients 7 en forme de boîte, sous les ordres d'un dispositif informatique 40.

Ce dispositif comporte, dans sa mémoire, des protocoles d'opération, un protocole pour chaque type d'opération, qui indique les instruments devant être utilisés au cours de l'opération, le cas échéant dans leur ordre d'utilisation. Etant donné qu'à chaque type d'opération correspond un boîtier 7, les instruments devant être rangés dans cette boîte sont indiqués par le protocole établi pour cette opération. A cette fin, le dispositif informatique 40 identifie tout d'abord, d'après le signal qu'il vient de recevoir du lecteur 38 du poste de reconnaissance, le type de l'instrument qui vient d'être examiné et détermine, en se reportant aux différents protocoles à quel type d'opération et ainsi à quelle boîte 7 un instrument de ce type est destiné. Puis, il donne l'ordre au dispositif de rangement 6 de saisir l'instrument identifié dans le plateau et de le ranger dans la boîte appropriée.

En comparant les instruments rangés dans une boîte à ceux figurant dans le protocole, le dispositif informatique connaît à tout moment l'état de "remplissage" de chaque boîte 7. S'il constate qu'une boîte est terminée, c'est-à-dire contient tous les instruments nécessaires pour l'opération considérée, la boîte est fermée, par exemple par la mise en place de son couvercle.

L'installation prévoit également la possibilité d'écarter des instruments jugés non aptes à être utilisés, du circuit d'utilisation, en les plaçant dans une boîte de rebus 8. Diverses raisons pourraient motiver cette mesure, par exemple l'usure d'un instrument, l'impossibilité de l'identifier ou parce qu'il s'agit d'un instrument souillé.

Concernant le poste de rangement 6, des appareils susceptibles de fonctionner de la manière décrite plus haut, sont largement connus, si bien qu'il n'est pas nécessaire de décrire l'appareil utilisé dans le cadre de l'invention, précisément. Il convient d'indiquer qu'un tel appareil comporte un bras robot capable de saisir les instruments dans leur plateau et de les placer ensuite, en fonction des instructions reçues du dispositif informatique, dans les boîtes appropriées.

Concernant le fonctionnement de l'installation et le déroulement du procédé ainsi que les différentes étapes de celui-ci, ils découlent de la description qui vient d'être faite. Il va sans le préciser davantage, que pour chaque transfert d'un plateau sur le convoyeur, celui-ci est arrêté pendant un bref instant de temps nécessaire à la pose du plateau. Les arrêts du convoyeur pour le chargement des plateaux et le processus de reconnaissance des instruments par le poste de reconnaissance ainsi que le rangement des instruments sont coordonnés par le dispositif informatique.

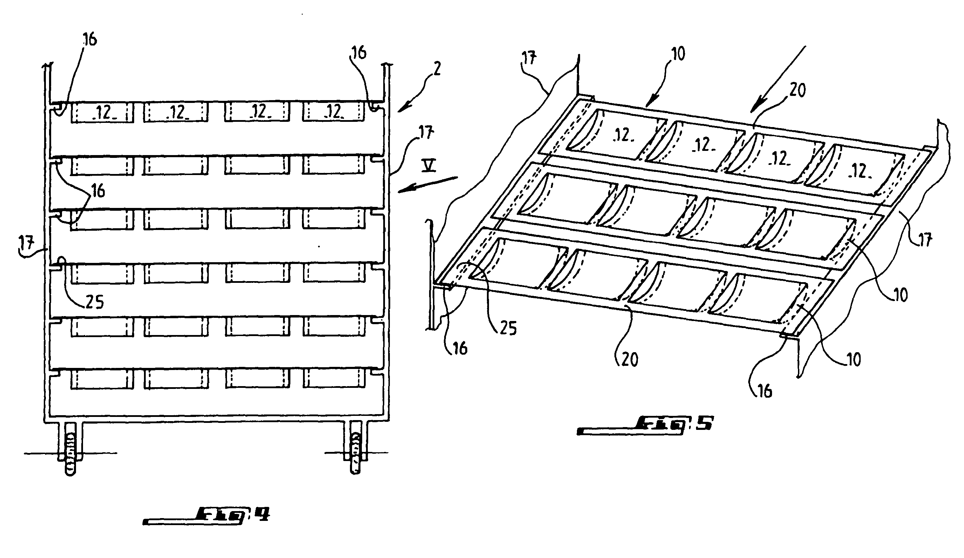

La description de l'invention, qui vient d'être faite, n'a été donnée qu'à titre d'exemple et des multiples modifications peuvent être apportées sans sortir du cadre de l'invention. Pour augmenter la capacité de stockage du chariot 2, chaque plateau 10 pourrait comporter quatre alvéoles, comme le montrent les figures 4 et 5. La flèche symbolise l'action du mécanisme de déplacement du plateau vers le convoyeur 3.

Il est à noter que l'installation et le support des instruments ont été décrits pour faciliter la compréhension de l'invention. Ils ne font pas partis de la présente invention et font les objets de demandes de brevets parallèles bénéficiant de la même date de priorité que la présente invention.

## Revendications

1. Instrument tel qu'un instrument chirurgical d'un type prédéterminé, défini par sa forme et sa fonction, parmi une pluralité d'instruments de types différents , et pourvu d'un insert (35) porteur d'un code d'identification de l'instrument (14), disposé dans une cavité fermée préalablement pratiquée dans l'instrument (14) **caractérisé en ce que** l'insert (35) est réalisé en un matériau relativement opaque aux rayons X et l'instrument est en un matériau relativement transparent à ces rayons X, pour qu'il puisse être reconnu à l'aide des rayons X.

2. Instrument selon la revendication 1, **caractérisé en ce que** les éléments de code de l'insert (35) sont constitués par des éléments tels que la forme spécifique de l'insert, la forme d'une encoche dans l'insert ou des marques de celui-ci.

3. Instrument selon la revendication 1 ou 2, l'insert est réalisé en un matériau tel que du laiton ou un alliage à base de laiton, alors que l'instrument est en acier inoxydable.

## Claims

1. An instrument, such as a surgical instrument, of a predetermined type, defined by the shape and function thereof, among a plurality of instruments of different types, and provided with an insert (35) bearing a code identifying the instrument (14), arranged inside a closed cavity previously made within the instrument (14), **characterized in that** the insert (35) is made of a material, which relatively opaque to X-rays, and the instrument consists of a material, which is relatively transparent to such X-rays, so that it can be recognized by means of the X-rays.

2. The instrument according to claim 1, **characterized in that** the code items of the insert (35) are composed of items such as the specific shape of the insert, the shape of a notch in the insert, or marks thereon.

3. The instrument according to claim 1 or 2, wherein the insert is made of a material such as brass or an alloy based on brass, whereas the instrument consists of stainless steel.

## Patentansprüche

1. Instrument, wie etwa ein chirurgisches Instrument, vorherbestimmter Art, das durch seine Form und Funktion definiert wird, aus einer Vielzahl von verschiedenartigen Instrumenten, und das mit einem Einsatzteil (35) versehen ist, das einen Code zur Identifizierung des Instruments (14) trägt, in einem geschlossenen Hohlraum angeordnet ist, der zuvor in dem Instrument (14) eingerichtet wurde, **dadurch gekennzeichnet, dass** das Einsatzteil (35) aus einem Material ausgebildet wird, das für Röntgenstrahlen relativ undurchsichtig ist, und das Instrument aus einem Material besteht, das für diese Röntgenstrahlen relativ transparent ist, damit es anhand der Röntgenstrahlen erkannt werden kann.

2. Instrument nach Anspruch 1, **dadurch gekennzeichnet, dass** die Code-Elemente des Einsatzteils (35) aus Elementen bestehen wie etwa der spezifischen Form des Einsatzteils, der Form einer Einkerbung in dem Einsatzteil oder Markierungen darauf.

3. Instrument nach Anspruch 1 oder 2, wobei das Einsatzteil aus einem Material wie etwa Messing oder einer Legierung auf Messingbasis ausgebildet ist, während das Instrument aus rostfreiem Stahl besteht.
